# FASCICULE DE BREVET EUROPEEN

(11) **EP 2 616 016 B1**
(45) Date de publication et mention de la délivrance du brevet: **27.04.2016**
(21) Numéro de dépôt: 11773092.9
(22) Date de dépôt: 15.09.2011
(51) Int. Cl.: A61F 2/30, A61B 17/86, A61F 2/36, A61F 2/40

(54) **ANNEAU D'ESPACEMENT ET DE FIXATION POUR CUPULE D'ÉPAULE**
DISTANZ- UND FIXIERRING FÜR EINE SCHULTERPFANNE
SPACING AND FIXING RING FOR SHOULDER CUP

(30) Priorité: 17.09.2010 FR 1057443
(43) Date de publication de la demande: 24.07.2013
(73) Titulaire: 3S Ortho, 69009 Lyon (FR)
(72) Inventeur: DUPORT, Marc, 31400 Toulous (FR)
(74) Mandataire: Jeannet, Olivier
(86) Numéro de dépôt international: PCT/FR2011/052118
(87) Numéro de publication internationale: WO 2012/035266

(56) Documents cités:
- EP-A2- 1 782 764
- WO-A1-00/62718
- WO-A1-01/24737
- FR-A1- 2 674 122

## Description

La présente invention se rapporte à un dispositif pour l'espacement et la fixation d'une prothèse d'épaule.

En France, environ 15 000 prothèses d'épaule sont posées par an, incluant les prothèses totales d'épaules, dites standards, les prothèses inversées, et plus récemment les prothèses de resurfaçage.

Ce chiffre, en nette augmentation ces dernières années, est en particulier dû à l'augmentation du nombre de pathologies pour lesquelles la pose d'une prothèse d'épaule est maintenant indiquée.

Dans ce contexte, la chirurgie prothétique de l'épaule reste une chirurgie très spécialisée, de haute technicité, nécessitant un apprentissage long et l'utilisation d'ancillaires souvent complexes. Une simplification de ces ancillaires est donc souhaitable afin de faciliter le geste opératoire, de le rendre plus reproductible et donc de diminuer les complications postopératoires. Une telle simplification de l'ancillaire et du geste opératoire aurait également l'avantage de diminuer les coûts de ces opérations de pose de prothèses d'épaule.

Il existe plusieurs types de prothèses d'épaule. Certaines prothèses classiques telles que celle décrite dans US 2004/153161 sont fixées sur l'humérus, grâce à une tige humérale, après une résection conséquente de l'os. L'implantation d'une tige humérale nécessite un évidement important dans l'os pour l'accueillir, diminuant ainsi le capital osseux et fragilisant l'os. La pose d'une telle prothèse est longue et complexe. Après installation de la tige dans l'évidement, l'espace entre l'os et la tige humérale est généralement comblé avec du ciment. Avec le temps, le ciment peut se disloquer et ne plus assurer alors une fixation correcte de la tige.

Il existe également des prothèses de resurfaçage, telles que celle décrite dans FR 2 928 827, qui sont posées sur l'épiphyse de l'humérus après un éventuel ponçage léger de la surface de l'épiphyse. De telles prothèses ne sont pas nécessairement ancrées dans l'os comme décrit précédemment.

Lorsqu'un simple resurfaçage de l'épiphyse n'est pas indiqué, par exemple en cas de fragilité osseuse trop importante, le chirurgien peut choisir de mettre en oeuvre une technique intermédiaire dite de « demi-resurfaçage » ou une partie de l'épiphyse est réséquée. C'est également le cas lorsque la pose d'une glène prothétique est indiquée et qu'une résection importante de l'extrémité de l'épiphyse doit être effectuée pour libérer l'espace nécessaire à la mise en place de la glène prothétique. Le chirurgien est alors amené à utiliser une prothèse telle que celle décrite dans le document EP 0538 895, comportant une cupule destinée à remplacer la partie de l'épiphyse réséquée et une vis d'ancrage destinée à être fixée dans l'os par vissage, une telle vis d'ancrage comportant à cet effet un filetage sur toute sa longueur. La cupule est alors fixée sur la vis d'ancrage.

Lorsque la cupule utilisée est creuse, et lorsque la partie réséquée de l'os est importante, il peut être nécessaire de recourir à des éléments d'espacement disposés entre la cupule et la vis d'ancrage. De tels éléments d'espacement sont décrits, par exemple, dans la demande EP 1 470 802. Ceux-ci ne contribuent pas à l'ancrage de la cupule. Par ailleurs, les éléments d'espacement ne permettent pas la fixation de la cupule et nécessitent une vis d'ancrage.

Les documents EP 1 782 764 A1 et WO 01 24737 A1 décrivent d'autres prothèses existantes. Le préambule de la revendication 1 est basé sur ce que décrit le premier de ces documents.

La présente invention a pour objet de remédier en tout ou partie aux différents inconvénients cités précédemment.

Dans ce contexte, la présente invention a pour objet de proposer un dispositif pour l'espacement et la fixation d'une cupule qui permet d'ajuster la position de la cupule en cas de résection importante de l'os tout en assurant un ancrage correcte de la cupule dans l'os.

A cet effet, la présente invention se rapporte à un dispositif pour l'espacement et la fixation d'une prothèse selon la revendication 1.

Ainsi, un dispositif selon l'invention permet d'ajuster la position d'une cupule comportant une calotte sphérique creuse par rapport à l'extrémité réséquée de l'os et permet de fixer la cupule. En effet la première face de la portion intermédiaire d'appui offre une surface d'appui sur laquelle la cupule peut être placée en appui. L'épaisseur de la portion d'appui peut ainsi être adaptée pour espacer la cupule de l'os d'une distance adaptée au patient. Cette portion d'appui est particulièrement avantageuse lorsque l'os est fragilisé puisqu'elle permet d'éviter que la cupule ne soit directement placée en appui sur l'os et ne le fragilise d'avantage. La portion de montage s'accouple avec une cupule de façon simple et rapide tandis que la portion de fixation permet de fixer le dispositif dans l'os, une vis d'ancrage n'étant pas indispensable : la pose de la cupule est donc facilitée.

De plus, le dispositif selon l'invention permet au chirurgien de combler un espace important laissé vacant par une résection importante de la tête humérale, en cas par exemple de pose d'une glène prothétique, et d'ajuster au mieux le positionnement et l'espacement de la cupule avec la glène prothétique.

Selon l'invention, la portion intermédiaire d'appui est sensiblement circulaire. Une portion d'appui sensiblement circulaire est particulièrement adaptée à une cupule comportant une calotte sphérique.

Selon une possibilité, la portion intermédiaire d'appui présente un bord libre formant une portion de calotte sphérique.

Selon une caractéristique de l'invention, la portion intermédiaire d'appui présente un orifice traversant ménagé au centre de ladite portion intermédiaire d'appui. L'orifice traversant permet d'utiliser une cupule présentant un plot central tronconique s'étendant depuis le pôle de la cupule pour permettre de fixer directement ladite cupule dans l'os grâce à une vis d'ancrage et ainsi améliorer la fixation de la cupule.

Selon une possibilité, la portion de montage présente une surface extérieure présentant sensiblement une forme tronconique mâle. Une forme tronconique mâle est particulièrement adaptée pour permettre le montage d'une cupule présentant un logement tronconique femelle complémentaire, un tel montage étant simple et rapide à effectuer durant une opération.

Dans un mode d'exécution, la portion de montage présente une surface intérieure présentant sensiblement une forme tronconique femelle. Une telle forme tronconique femelle permet de monter un éventuel anneau complémentaire, tel que ceux décrits dans la demande de brevet EP 1 470 802, pour espacer d'avantage la cupule.

Selon une possibilité, la surface extérieure de la portion de fixation comporte au moins une rainure formant harpon.

Selon une caractéristique, l'extrémité libre de la portion de fixation est crénelée. Une telle extrémité libre crénelée évite la rotation du dispositif lorsqu'il est fixé dans l'os.

Selon une possibilité, la portion de fixation présente une paroi tubulaire dont l'épaisseur diminue en éloignement de la portion intermédiaire d'appui. La diminution de l'épaisseur de la paroi facilite l'insertion de la portion de fixation dans l'os.

Avantageusement, les pattes de l'extrémité de fixation, disposées entre les créneaux, comportent des bords obliques dans le sens de la rotation, lors de l'impaction de la prothèse. Les bords sont inclinés de la même façon que des nervures ménagées sur la face interne de la cupule, et permettent d'imposer une rotation de 10° lors de l'impaction de la prothèse.

L'invention sera bien comprise à l'aide de la description détaillée qui est exposée ci-dessous en regard du dessin annexé, représentant à titre d'exemple non limitatif une forme de réalisation d'un dispositif, dans lequel :
La figure 1 est une vue en perspective d'une prothèse comportant un dispositif monté sur une cupule ;
la figure 2 est une vue en perspective du dispositif de la figure 1 ;
la figure 3 est une autre vue en perspective du dispositif de la figure 1.

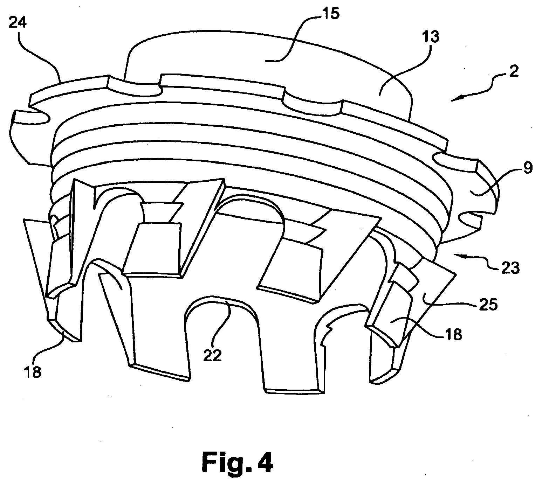

la figure 4 est une vue en perspective d'une variante du dispositif de figure 3.

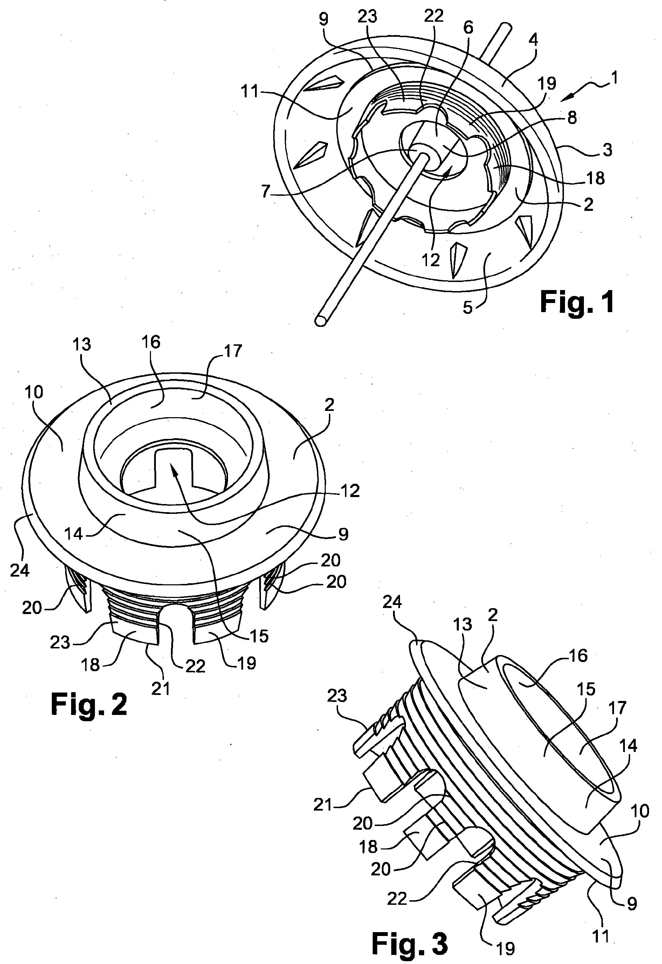

Une prothèse 1, illustrée à la figure 1, comporte un dispositif 2 et une cupule 3 destinée à être montée dans le dispositif 2.

La cupule 3 présente une calotte sphérique 4 creuse, présentant une face intérieure 5 et un plot central 6 tronconique faisant saillie du centre de la face intérieure 5 de la calotte sphérique 4. Le plot central 6 délimite une portion de montage mâle 7 formant un cône morse mâle 8.

Le dispositif 2 pour l'espacement et la fixation, illustré aux figures 1 à 3, est destiné à être disposé entre la cupule 3 et un os, notamment une épiphyse humérale. Le dispositif 2 est destiné à être fixé dans cet os et comporte une portion intermédiaire d'appui 9, illustrée aux figures 1 à 3, présentant une première face 10, illustrée à la figure 2, et une deuxième face 11, opposée à la première face 10, comme illustrée aux figurés 1 et 3. La première face 10 est destinée à être disposée en regard de la cupule 3 et la deuxième face 11 est destinée à être disposée en regard de l'os. La portion intermédiaire d'appui 9 est sensiblement circulaire afin de coopérer avec la face intérieure 5 de la calotte sphérique 4. Avantageusement, le bord libre 24 de la surface d'appui 9 est arrondi, formant une portion de calotte sphérique dimensionnée pour épouser la forme de la face intérieure 5 lorsque le dispositif 2 est disposé dans la calotte sphérique 4. La portion intermédiaire d'appui 9 présente un orifice traversant 12 ménagé au centre de ladite portion intermédiaire d'appui 9 pour le passage du plot central 6 tronconique.

Le dispositif 1 comporte en outre une portion de montage 13 cylindrique creuse, illustrée aux figures 2 et 3, qui s'étend à partir de la première face 10 et qui est conçue pour coopérer avec une portion de montage complémentaire, non représentée, de la cupule 3. La portion de montage 13 présente une surface extérieure 14 présentant sensiblement une forme tronconique mâle. Avantageusement la surface extérieure 14 de la portion de montage 13 forme un cône morse mâle 15. Ce cône morse mâle 15 est dimensionné pour coopérer avec un logement tronconique femelle, non représenté, ménagé sur la face intérieure 5 de la calotte sphérique 4 et ainsi assurer le montage du dispositif 2 sur la cupule 3. Avantageusement, la portion de montage 13 comporte également une surface intérieure 16 qui présente sensiblement une forme tronconique femelle. Avantageusement la surface intérieure 16 forme un cône morse femelle 17 conçu pour recevoir, par exemple, un cône morse mâle d'un anneau d'espacement connu.

Le dispositif 2 comporte également une portion de fixation 18 cylindrique creuse s'étendant à partir de la deuxième face 11. Cette portion de fixation 18 est conçue pour fixer le dispositif 2 dans l'os. La surface extérieure 19 de la portion de fixation 18 comporte une pluralité de rainures 20 formant harpon destinées à coopérer avec l'os pour assurer une fixation adéquate du dispositif 2 et pour offrir une bonne résistance à l'arrachement. En outre, l'extrémité libre 21 de la portion de fixation 18 comporte des créneaux 22, illustrés aux figures 2 et 3. De tels créneaux 22 présentent, par exemple, une forme arrondie, comme dans le mode d'exécution représenté sur les figures. De manière alternative, les créneaux 22 peuvent présenter une forme rectangulaire. Les créneaux 22 améliorent la fixation dans l'os de la portion de fixation 18 et empêchent la rotation du dispositif 2. Enfin, la portion de fixation 18 présente une paroi tubulaire 23 dont l'épaisseur diminue en éloignement de la portion intermédiaire d'appui 9.

Les pattes de l'extrémité de fixation 18, disposées entre les créneaux 22, comportent des bords obliques 25, dans le sens de la rotation pour favoriser la rotation du dispositif, lors de l'impaction de la prothèse.

Avantageusement, le dispositif 2 est en totalité ou en partie recouvert d'un revêtement en matériau favorisant la repousse osseuse tel que de l'hydroxyapatite.

Ainsi, le dispositif 2 selon l'invention décrit ci-dessus permet d'ajuster la position de la cupule 3 comportant une calotte sphérique 4 par rapport à l'extrémité réséquée de l'os et permet de fixer de la cupule 3. L'épaisseur de la portion intermédiaire d'appui 9 peut ainsi être adaptée pour espacer la cupule 3 de l'os d'une distance adaptée au patient. Cette portion intermédiaire d'appui 9 est particulièrement avantageuse lorsque l'os est fragilisé puisqu'elle évite que la cupule 3 ne soit directement placée en appui sur l'os et ne le fragilise d'avantage. La portion de montage 13 s'accouple avec la cupule 3 de façon simple et rapide tandis que la portion de fixation 18 fixe le dispositif 2 et la cupule 3 dans l'os.

Bien entendu, les exemples de réalisation évoqués ci-dessus ne présentent aucun caractère limitatif et d'autres détails et améliorations peuvent être apportés au dispositif 2 selon l'invention telle que définie dans les revendications.

## Revendications

1. Dispositif (2) pour l'espacement et la fixation d'une prothèse (1) d'épaule, le dispositif étant destiné à être disposé entre une cupule (3) et une épiphyse humérale, et destiné à être fixé dans cet os, **caractérisé en ce que** le dispositif (2) comporte trois portions d'une même pièce monobloc:
- une portion intermédiaire d'appui (9) présentant une première et une deuxième faces (10, 11) opposées, la première face (10) étant destinée à être disposée en regard de la cupule (3) et la deuxième face (11) étant destinée à être disposée en regard de l'os ,
- une portion de montage (13) cylindrique creuse s'étendant à partir de la première face (10) et conçue pour coopérer avec une portion de montage complémentaire de la cupule (3) ;
- une portion de fixation (18) cylindrique creuse s'étendant à partir de la deuxième face (11) et conçue pour fixer le dispositif (2) dans l'os ; ladite portion intermédiaire d'appui (9) étant sensiblement circulaire et adaptée à la cupule (3) comportant une calotte sphérique (4).

2. Dispositif (2) selon la revendication 1, **caractérisée en ce que** la portion intermédiaire d'appui (9) présente un bord libre (24) formant une portion de calotte sphérique.

3. Dispositif (2) selon la revendication 1 ou la revendication 2, **caractérisée en ce que** la portion intermédiaire d'appui (9) présente un orifice traversant (12) ménagé au centre de ladite portion intermédiaire d'appui (9).

4. Dispositif (2) selon l'une des revendications 1 à 3, **caractérisée en ce que** la portion de montage (13) présente une surface extérieure (14) présentant sensiblement une forme tronconique mâle.

5. Dispositif (2) selon l'une des revendications 1 à 4, **caractérisée en ce que** la portion de montage (13) présente une surface intérieure (16) présentant sensiblement une forme tronconique femelle.

6. Dispositif (2) selon l'une des revendications 1 à 5, **caractérisée en ce que** la surface extérieure (19) de la portion de fixation (18) comporte au moins une rainure (20) formant harpon.

7. Dispositif (2) selon l'une des revendications 1 à 6, **caractérisée en ce que** l'extrémité libre (21) de la portion de fixation (18) est crénelée.

8. Dispositif (2) selon l'une des revendications 1 à 7, **caractérisée en ce que** la portion de fixation (18) présente une paroi tubulaire (23) dont l'épaisseur diminue en éloignement de la portion intermédiaire d'appui (9).

## Patentansprüche

1. Vorrichtung (2) zur Distanzierung und Fixierung einer Schulterprothese (1), wobei die Vorrichtung dazu bestimmt ist, zwischen einer Pfanne (3) und einer Oberarmepiphyse angeordnet zu werden, und dazu bestimmt ist, in diesem Knochen fixiert zu werden, **dadurch gekennzeichnet, dass** die Vorrichtung (2) drei Abschnitte ein und desselben einstückigen Teils umfasst:
- einen Zwischenauflageabschnitt (9), der eine erste und eine zweite Fläche (10, 11) aufweist, die einander gegenüberliegen, wobei die erste Fläche (10) dazu bestimmt ist, gegenüber der Pfanne (3) angeordnet zu werden, und wobei die zweite Fläche (11) dazu bestimmt ist, gegenüber dem Knochen angeordnet zu werden;
- einen zylindrischen, hohlen Einbauabschnitt (13), der sich ausgehend von der ersten Fläche (10) erstreckt und ausgelegt ist, um mit einem komplementären Einbauabschnitt der Pfanne (3) zusammenzuwirken;
- einen zylindrischen, hohlen Fixierungsabschnitt (18), der sich ausgehend von der zweiten Fläche (11) erstreckt und ausgelegt ist, um die Vorrichtung (2) im Knochen zu fixieren;
wobei der Zwischenauflageabschnitt (9) im Wesentlichen rund und an die Pfanne (3) angepasst ist, die eine Kugelkalotte (4) umfasst.

2. Vorrichtung (2) nach Anspruch 1, **dadurch gekennzeichnet, dass** der Zwischenauflageabschnitt (9) einen freien Rand (24) aufweist, der einen Kugelkalottenabschnitt bildet.

3. Vorrichtung (2) nach Anspruch 1 oder 2, **dadurch gekennzeichnet, dass** der Zwischenauflageabschnitt (9) eine Durchgangsöffnung (12) aufweist, die in der Mitte des Zwischenauflageabschnitts (9) ausgeführt ist.

4. Vorrichtung (2) nach einem der Ansprüche 1 bis 3, **dadurch gekennzeichnet, dass** der Einbauabschnitt (13) eine Außenfläche (14) aufweist, die im Wesentlichen eine kegelstumpfförmige Steckerform aufweist.

5. Vorrichtung (2) nach einem der Ansprüche 1 bis 4, **dadurch gekennzeichnet, dass** der Einbauabschnitt (13) eine Innenfläche (16) aufweist, die im Wesentlichen eine kegelstumpfförmige Buchsenform aufweist.

6. Vorrichtung (2) nach einem der Ansprüche 1 bis 5, **dadurch gekennzeichnet, dass** die Außenfläche (19) des Fixierungsabschnitts (18) mindestens eine hakenförmige Nut (20) aufweist.

7. Vorrichtung (2) nach einem der Ansprüche 1 bis 6, **dadurch gekennzeichnet, dass** das freie Ende (21) des Fixierungsabschnitts (18) gezackt ist.

8. Vorrichtung (2) nach einem der Ansprüche 1 bis 7, **dadurch gekennzeichnet, dass** der Fixierungsabschnitt (18) eine röhrenförmige Wand (23) aufweist, deren Dicke mit zunehmender Entfernung vom Zwischenauflageabschnitt (9) abnimmt.

## Claims

1. Device (2) for spacing and securing a shoulder prosthesis (1) long, the device being intended to be placed between a cup (3) and a humeral epiphysis, and being intended to be fixed in the bone, **characterized in that** the device (2) comprises three portions of a same single part:
- an intermediate supporting portion (9) having opposing first and second faces (10, 11), the first face (10) being intended to be placed facing the cup (3) and the second face (11) being intended to be placed facing the bone;
- a hollow cylindrical assembling portion (13) extending from the first face (10) and adapted to cooperate with a complementary assembling portion of the cup (3);
- a hollow cylindrical fixing portion (18) extending from the second face (11) and adapted for securing the device (2) in the bone;
said intermediate supporting portion (9) being substantially circular and adapted to the cup (3) having a spherical cap (4).

2. Device (2) according to claim 1, **characterized in that** the intermediate supporting portion (9) has a free edge (24) forming a portion of a spherical cap.

3. Device (2) according to claim 1 or claim 2, **characterized in that** the intermediate supporting portion (9) has a through hole (12) formed at the center of said intermediate supporting portion (9).

4. Device (2) according to one of claims 1 to 3, **characterized in that** the assembling portion (13) has an outer surface (14) having substantially a frustoconical male shape.

5. Device (2) according to one of claims 1 to 4, **characterized in that** the assembling portion (13) has an inner surface (16) having substantially a frustoconical female shape.

6. Device (2) according to one of claims 1 to 5, **characterized in that** the outer surface (19) of the fixing portion (18) has at least one groove (20) forming a harpoon.

7. Device (2) according to one of claims 1 to 6, **characterized in that** the free end (21) of the fixing portion (18) is crenellated.

8. Device (2) according to one of claims 1 to 7, **characterized in that** the fixing portion (18) has a tubular wall (23) whose thickness decreases away from the intermediate supporting portion (9).
